# EUROPEAN PATENT SPECIFICATION

(11) **EP 1 156 778 B1**
(45) Date of publication and mention of the grant of the patent: **14.07.2004**
(21) Application number: 00913571.6
(22) Date of filing: 23.02.2000
(51) Int. Cl.: A61K 7/32, A61K 7/36

(54) **DEODORANT COMPOSITIONS**
DEODORANTZUSAMMENSETZUNGEN
COMPOSITIONS DESODORISANTES

(30) Priority: 26.02.1999 GB 9904557
(43) Date of publication of application: 28.11.2001
(73) Proprietor: THE GILLETTE COMPANY, Boston, Massachusetts 02199 (US)
(72) Inventor: CAUSTON, Brian Edward, Reading, Berkshire RG1 4QX (GB); TAVERN, Sydney Christopher, Reading, Berkshire RG2 0DX (GB)
(74) Representative: Ebner von Eschenbach, Jennifer
(86) International application number: PCT/US2000/004497
(87) International publication number: WO 2000/049996

(56) References cited:
- EP-A- 0 333 118
- EP-A- 0 459 003
- US-A- 5 407 743

## Description

This invention relates to deodorant compositions.

Zinc oxide is known to have bactericidal properties, and has been widely used in deodorant compositions. It is believed to work by the zinc ions of the zinc oxide going into solution in the outer layer of the skin, where they kill microorganisms by preventing enzymes therein from functioning properly.

Inhalation of zinc oxide particles smaller than about 10 micrometers can cause adverse reactions in humans and animals. For this reason, many countries have adopted Health and Safety legislation forbidding the use of zinc oxide particles smaller than 10 micrometers in aerosols. This places a lower limit on the size of zinc oxide particles that can be used in aerosol deodorant compositions.

Although zinc oxide particles can easily be made to a size greater than 10 micrometers, such particles appear as a white powder and are considered unsightly. In order to avoid this white appearance it would be necessary to use a zinc oxide particle size below 10 micrometers, but this would not be permitted under the legislation referred to above.

We have now devised some deodorant compositions containing zinc oxide, or a zinc salt, whereby one or more of a number of advantages over prior known compositions can be obtained including, if desired, the advantage of substantially transparent zinc oxide (or zinc salt) without the need to use particles having a size below 10 micrometers.

According to one aspect of the invention, we provide a deodorant composition for topical application, which comprises a plurality of particles which each comprise a glass microsphere at least partially coated with a zinc compound sintered to the glass microsphere, the zinc compound being selected from zinc oxide, a zinc salt or any mixture of two or more thereof, said particles being dispersed in a carrier.

Spherical particles comprising a zinc oxide coating are known. Thus, US-A-5407743 describes electromechanical devices such as transducers which comprise glass spheres of size 1 to 500 microns coated with zinc oxide. The zinc oxide coating is formed by applying a zinc oxide precursor to the spheres and then contacting the coated spheres with an oxidizing agent to form a zinc oxide coating on the spheres.

J. Soc. Cosmet. Chem., 41, 197-207 (May/June 1990) describes a mechanochemical process wherein fine particle zinc oxide is mixed with spherical resin cores to provide a hybrid powder of cores with their surfaces uniformly covered with zinc oxide. The powder has deodorizing qualities.

EP-A-0 459 003 discloses a coating composition and an antimicrobial deodorant composed of the coating composition, comprising 5 to 45 parts by weight of specific organoalkoxysiloxane, hydrolysate of alkoxysiloxane and partial condensates thereof; 0.3 to 40 parts by weight of a filler; 0.03 to 3 parts by weight of an antimicrobial metal comprising at least a metal salt selected from the group of silver, copper and zinc salts; 3 to 60 parts by weight of water; 5 to 80 parts by weight of alcohol, coated on a granular material selected from the group consisting of particles of sand, rubble stone, shell, glass and baked inorganic clay matter having a particle size of 0.1 to 30 mm in average diameter.

In the compositions of the present invention, the microspheres may be coated only with zinc oxide, or with both zinc oxide and a zinc salt, or with a salt alone (mixtures of two or more salts can be used). We prefer that the coating comprises zinc oxide and a salt since then the bactericidal activity of the zinc oxide is enhanced and the product remains active for an extended period. If the coating consists only of one or more zinc salts with no zinc oxide present, the active bactericidal life of the coating is usually relatively short. If the coating consists of zinc oxide alone, its bactericidal activity is not as great as when a zinc salt is also present.

The preferred zinc salts are those which can be made by reacting a zinc oxide coating with an acid to form the salt *in situ* on the microspheres. Preferably, the zinc salts are only sparingly soluble in water so that they are not quickly removed by contact with water. The preferred salts are made by reacting the zinc oxide with a carboxylic acid or a substituted phenol. Examples of the preferred salts are the acetate, pidolate, pyrrolidone-5-carboxylate, cinnamate, citrate and glycinate, but these are merely illustrative of the many possible salts which can advantageously be used.

The compositions according to the invention may also contain at least one further bactericide in addition to the zinc oxide or zinc salt.

The colour of the particles depends upon the colour of the glass microsphere core, because the layer of the zinc compound (by which we mean zinc oxide and/or one or more zinc salts) will be substantially transparent, provided that it is thin enough. This makes it possible to provide a deodorant composition which has the bactericidal advantages of zinc oxide without the white appearance normally associated with deodorant compositions containing zinc oxide particles larger than 10 microns. In order to comply with legislation relating to the size of zinc oxide particles in aerosols, the glass microspheres would normally have a minimum diameter of about 10 micrometers, although for other uses, they may of course be smaller, eg. down to 5 micrometers or less. We have found that, in order to obtain good transparency, it is preferable that the diameter of the glass microspheres should not exceed about 50 micrometers. However, greater diameters can be used, for example up to 150 micrometers or more.

In order to prepare the zinc oxide coated microspheres of the invention, it is important to control the reaction temperature closely. In accordance with another aspect of the invention, we provide a method of coating glass microspheres with zinc oxide, which method comprises heating an agitated mixture of the glass microspheres and a zinc compound in a liquid reaction medium to form a milky suspension of colloidal zinc oxide, and further heating the suspension to 190°C to 200°C to deposit the zinc oxide as an adherent coating sintered on the glass microspheres. We have found that if the temperature is outside this range, then either the yield of zinc oxide or the adherence of the coating, or both, will be generally unsatisfactory.

We have also found that heating in two stages, at two different temperatures, provides the best combination of adhesion and yield. Accordingly, we prefer to form the milky suspension by heating the mixture to 140°C to 160°C, preferably for 1 to 3 hours. Then, we prefer to heat for a further 1 to 3 hours to deposit said coating at 190° to 200°C.

The method of the invention is carried out in a liquid reaction medium. This should have a high enough dielectric constant that it dissolves the zinc compound, and also a high enough boiling point to allow the high temperatures to be obtained to produce the zinc oxide and allow sintering of the coating on the surface of the glass microspheres. Among the preferred such solvents are the glycols, particularly (but not exclusively) diethylene glycol, tetraethylene glycol and poly(ethylene glycol). Diethylene glycol has a dielectric constant of 31.7 at 20°C and a boiling point of 245°C. Poly(ethylene glycol) of molecular weight 300 has a dielectric constant of 37.7 at 20°C and a boiling point of 198°C.

Any zinc compound can be used which will hydrolyse in the process to yield zinc oxide. The preferred zinc compounds are the carboxylates (other than the oxalate), most preferably the acetate. The chloride and sulphate are not used since zinc oxide is not readily formed therefrom in glycol solutions. As will be clear to those skilled in the art, the suitability of any particular zinc compound can be ascertained by routine trial and experiment.

When the zinc compound used is zinc acetate dihydrate, we prefer the glycol to be diethylene glycol, tetraethylene glycol or poly(ethylene glycol) of molecular weight 300 (PEG Mr 300). The use of these glycols leads to the formation of the zinc oxide as a single phase which is preferred. Other glycols can also be used although some may lead to the formation of the less desirable impure multiphase compounds. In general, any glycol can be used but we prefer to use one of the above three or any mixture of two or all three thereof.

According to a further feature of the invention, when it is desired that the coating on the microspheres should comprise a zinc salt, the salt can be formed *in situ* by reacting the zinc oxide coating with an acid. We prefer to form a layer of a zinc salt at the exposed surface of the zinc oxide, so that the product comprises microspheres with a zinc oxide coating having zinc salt thereon. However, if desired, the whole of the zinc oxide can be reacted with acid so that the product then comprises microspheres with zinc salt coatings (and no zinc oxide).

The zinc oxide coating of the invention comprises submicron primary particles or crystallites of the zinc oxide with diameters in the region of, for example, about 30nm. These colloidal particles aggregate and form particulate clusters with diameters in the range of about 0.3 to about 0.5 micrometers. The size of the primary particles can be controlled by varying the reaction temperature as described more fully hereinafter.

It is usually preferred for the compositions of the invention to be transparent, in which case the glass microspheres are preferably substantially transparent. However, there may be circumstances in which it is desirable for the composition to have a particular colour. It is possible to provide the composition with a desired colour by using glass microspheres having the desired colour.

The compositions according to the invention may be provided in any convenient form suitable for topical application. For example, the compositions may be provided in the form of an aerosol, roll-on, gel, stick, cream, lotion or pump spray formulation.

In the compositions of the invention, the carrier is preferably a dermatologically acceptable vehicle such as, for example, a polyhydric alcohol, a silicone, ethanol, water etc. or any mixture of two or more thereof.

The compositions of the invention may be formulated into topical compositions such as aerosols, pump sprays, roll-ons, lotions, creams, gels, sticks etc. In particular, aqueous suspensions of the zinc oxide particles and salts may be directly utilized in oil-in-water and water-in-oil emulsions, such as the currently popular clear gel formulations, or in other aqueous based compositions such as aqueous based roll-ons. The compositions of the invention may be formulated into any known type of topical composition which utilizes powdered salts including, in particular, aerosol, liquid roll-on, cream and solid stick formulations in which the powdered salt is suspended in an anhydrous, dermatologically acceptable carrier, particularly a carrier comprising a silicone.

It will be appreciated that the precise formulation of the deodorant compositions will depend upon the type of deodorant composition which is desired. Thus, an aerosol formulation will contain predominantly a propellant, such as CAP30 propellant; a stick type formulation will typically contain predominantly propylene glycol; a roll-on formulation will typically contain predominantly cyclomethicone; and a gel formulation will typically contain predominantly water and propylene glycol. The deodorant compositions according to the invention may include any of the materials conventionally used in deodorant formulations. The compositions of the invention may comprise, in addition to the zinc oxide coated particles, other different particles.

The deodorant compositions according to the invention will contain sufficient zinc compound coated microspheres to reduce or prevent malodour when applied to the skin. Typically, they will contain from 0.1 to 25 wt% of the zinc compound coated microspheres. It is preferred that the compositions comprise 5 to 15 wt% of the zinc compound coated microspheres, more preferably up to about 10 wt%.

In accordance with another aspect of the invention, we provide an aerosol, roll-on, gel or pump spray device which includes a deodorant composition as described above.

According to another aspect of the invention we provide the use of particles comprising glass microspheres coated with a zinc compound as a deodorant in compositions for topical application. The particles may have the features of the particles of the deodorant composition described above.

Reference is now made to the accompanying drawings, in which:
Fig. 1 is a graph of log [colony forming units per cm²] versus time for coryneform bacteria; and
Fig. 2 is a graph of log [colony forming units per cm²] versus time for staphylococci bacteria.

The invention will now be described with reference to the following Examples.

### EXAMPLE 1

Zinc acetate dihydrate (44g) and sodalime glass microspheres (22g) were added to a I litre reaction kettle containing 500 ml of diethylene glycol. The sodalimc glass microspheres were obtained from Croxton & Gary under the trade name Spheriglass 2000 cpo, and had a diameter in the range 10 to 50 micrometers.

A flanged lid with a propeller type stirring paddle was placed over the reaction kettle. The kettle was then placed in an oil bath and subjected to a heating cycle of 2 hours at 150°C followed by 2 hours at 190°C. On completion of the heating, the kettle was removed from the oil bath and the diethylene glycol was decanted off. The remaining zinc oxide coated glass microspheres were filtered and washed in ethanol. The coated microspheres were then dried at room temperature.

A sample of the microspheres was coated with gold, then subjected to SEM analysis. This showed that the zinc oxide is present as clusters of primary particles.

These zinc oxide clusters consist of aggregates of much smaller zinc oxide primary particles. The primary particles are nm sized, and they are aggregated together to form the larger sub-micron sized (eg. 0.3-0.5 µm) clusters.

The zinc oxide coated microspheres prepared by this method can be used to form a variety of deodorant compositions, as exemplified in Examples 2 to 5.

### EXAMPLE 2

An aerosol type deodorant was prepared from the following materials:

| | |
|---|---|
| CAP30 propellant | 80 wt% |
| ZnO coated microspheres | 2.5 wt% |
| Ethanol | 11.5 wt% |
| Volatile silicone DC245 | 6 wt% |

### EXAMPLE 3

A stick type deodorant was prepared from the following materials:

| | |
|---|---|
| Water | 12 wt% |
| Propylene glycol | 71 wt% |
| Sodium stearate | 8 wt% |
| ZnO coated microspheres | 8 wt% |
| Perfume | 1 wt% |

### EXAMPLE 4

A suspension roll-on type deodorant was prepared from the following materials:

| | |
|---|---|
| Cyclomethicone | 86 wt% |
| ZnO coated microspheres | 8 wt% |
| Ethanol | 2.5 wt% |
| Quatemium 18 Hectorite | 2 wt% |
| Perfume | 1.5 wt% |

### EXAMPLE 5

A gel type deodorant was prepared from the following materials:

| | |
|---|---|
| Water | 25.25 wt% |
| Sorbitol | 14 wt% |
| Ethanol | 12 wt% |
| Propylene glycol | 22.5 wt% |
| ZnO coated microspheres | 8 wt% |
| Dimethicone (DC-225) | 10 wt% |
| Cyclomethicone & dimethicone copolyol | 8 wt% |
| Perfume | 0.25 wt% |

### EXAMPLE 6

A modified zinc oxide coating was prepared by reacting the ZnO coated microspheres with eugenol (4-allyl-2-methoxyphenol). The following formulation was prepared:

| | |
|---|---|
| Acetone | 89 wt% |
| Eugenol | 5 wt% |
| ZnO coated microspheres | 4 wt% |
| Distilled water | 2 wt% |

The formulation was stirred at room temperature for 48 hours, then the modified ZnO coated microspheres were isolated by filtration, washed and dried at room temperature.

### EXAMPLE 7

A modified zinc oxide coating was prepared by reacting the ZnO coated microspheres with pyrrolidone-5-carboxylic acid. The following formulation was prepared:

| | |
|---|---|
| Pyrrolidone-5-carboxylic acid | 5 wt% |
| ZnO coated microspheres | 4 wt% |
| Distilled water | 91 wt% |

The formulation was stirred at room temperature for 48 hours, then the modified ZnO coated microspheres were isolated by filtration, washed and dried at room temperature.

### EXAMPLE 8

An in-vivo method, known as the Williamson and Kligman surface scrub, was used to demonstrate the antimicrobial properties of the following five compositions:
(A) A control composition comprising ethanol/volatile silicone
(B) A composition comprising ethanol/volatile silicone and 0.3 wt% of a well known deodorant known as Triclosan (2,4,4'-trichloro-2-hydroxybiphenyl ether). This concentration is the highest amount of Triclosan that can be used without skin irritation.
(C) A composition according to the invention comprising ethanol/volatile silicone containing 2.5 wt% of the zinc oxide coated particles obtained by the method of Example 1.
(D) A composition according to the invention comprising ethanol/volatile silicone containing 10 wt% of the zinc oxide coated particles obtained by the method of Example 1.
(E) A composition according to the invention comprising ethanol/volatile silicone containing 20 wt% of the zinc oxide coated particles obtained by the method of Example 1.

In all the above compositions, the ratio of ethanol to silicone was 70/30, and there were substantially no other components present except those indicated.

The test was designed to measure the growth of the bacteria staphylococci and coryneforms in the axilla of each member of a group of participants. These two types of bacteria are primarily responsible for the development of odours.

Initially, a sample of bacteria was removed from the axilla of each participant. A 0.5 ml aliquot of various deodorant compositions was then applied to the axilla of the participants, and further bacteria samples were removed after 2, 7 and 24 hours. The level of coryneforms and staphylococci in each sample were then measured.

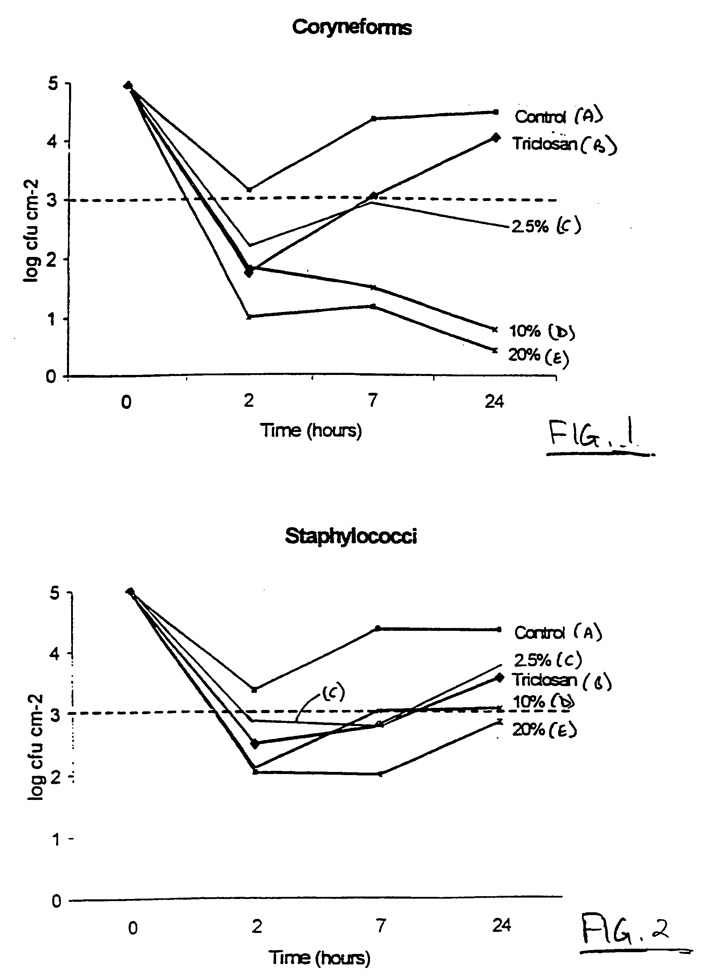

Bacteria level counts were obtained for each sample, and the change in bacteria levels with time is shown in Figs. 1 and 2. The black dotted line represents the level of bacteria at which odours start to become detectable. It is clear that compositions (C), (D) and (E) are substantially more effective than compositions (A) or (B). The results show that compositions (C), (D) and (E) were effective against coryneforms for up to 24 hours. Furthermore, the compositions (C), (D) and (E) did not cause any skin irritation.

## Claims

1. A deodorant composition for topical application, which comprises a plurality of particles which each comprise a glass microsphere at least partially coated with a zinc compound sintered to the glass microsphere, the zinc compound being selected from zinc oxide, a zinc salt or any mixture of two or more thereof, said particles being dispersed in a carrier.

2. A composition according to claim 1, wherein the coating comprises zinc oxide and a zinc salt, preferably the zinc salt is zinc pidolate, zinc acetate, zinc ougolinate, zinc pyrrolidone-5-carboxylate, zinc cinnamate, zinc citrate or zinc glycinate.

3. A composition according to claim 1 or 2, wherein the zinc salt is formed by contacting a zinc oxide coating on the microspheres with an acid, preferably a carboxylic acid or a phenol.

4. A composition according to any of claims 1 to 3, in which the glass microspheres:
(a) have at least one further bactericide in addition to the zinc oxide;
(b) have a diameter of from 5 to 150 micrometers; or
(c) have a diameter of from 10 to 50 micrometers.

5. A composition according to any of claims 1 to 4, wherein the zinc oxide or zinc salt is present in the coating in particle clusters of a diameter from 0.3 to 0.5 micrometers, preferably wherein said particles are essentially transparent.

6. A composition according to any of claims 1 to 5, wherein the carrier is a dermatologically acceptable vehicle, which preferably comprises a polyhydric alcohol, a silicone, ethanol, water or any combination of two or more thereof.

7. A composition according to claim 6, which contains from 0.01 to 10% by weight of said particles.

8. A composition according to any preceding claim, which is in the form of an aerosol, roll-on, gel, stick, cream, lotion or pump spray formulation.

9. The use of particles comprising glass microspheres having sintered thereto a coating of zinc oxide, a zinc salt or any mixture of two or more thereof, as a deodorant in compositions for topical application.

10. The use according to claim 9, wherein the particles are as defined in any of claims 2 to 5.

11. A method of preventing or reducing malodour which comprises applying to the skin a deodorant composition as claimed in any of claims 1 to 8.

12. A method of coating glass microspheres with zinc oxide, which method comprises heating an agitated mixture of glass microspheres, and a zinc compound in a liquid reaction medium, to form a milky suspension of colloidal zinc oxide, and further heating the suspension to 190°C to 200°C to deposit the zinc oxide as an adherent coating sintered on the glass microspheres.

13. A method according to claim 12, wherein:
(a) the mixture is heated to 140°C to 160°C to form said milky suspension;
(b) the liquid reaction medium comprises a glycol, preferably diethylene glycol or poly(ethylene glycol); and/or
(c) the mixture is heated for 1 to 3 hours to form said suspension, and for a further I to 3 hours to deposit said coating.

14. A method of coating glass microspheres with a zinc salt which comprises coating glass microspheres with zinc oxide by the method claimed in any of claims 12 to 13, and then contacting the coating with an acid to form thereon a zinc salt.

15. A method according to claim 14, wherein:
(a) only part of the zinc oxide coating is converted to a zinc salt;
(b) the acid is a carboxylic acid or a phenol;
(c) the acid is a carboxylic acid which is pidolic acid, acetic acid, euginol, pyrrolidone-S-carboxylic acid, cinnamic acid, citric acid or glycine; and/or
(d) after forming the zinc salt on the microspheres, they are washed with water without removal of the salt.

## Patentansprüche

1. Deodorantzusammensetzung zur topischen Aufbringung, die eine Mehrzahl von Partikeln aufweist, von denen jedes ein Glasmikrokügelchen aufweist, das mindestens teilweise mit einer Zinkverbindung beschichtet ist, die auf dem Glasmikrokügelchen aufgesintert ist, wobei die Zinkverbindung ausgewählt ist aus Zinkoxid, einem Zinksalz oder einer Mischung von zwei oder mehreren davon und wobei die Partikel in einem Träger dispergiert sind.

2. Zusammensetzung nach Anspruch 1, worin die Beschichtung Zinkoxid und ein Zinksalz aufweist, wobei das Zinksalz vorzugsweise Zinkpidolat ist, Zinkacetat, Zinkeugolinat, Zinkpyrrolidon-5-carboxylat, Zinkcinnamat, Zinkcitrat oder Zinkglycinat.

3. Zusammensetzung nach Anspruch 1 oder 2, worin das Zinksalz erzeugt wird durch Kontaktieren einer Zinkoxid-Beschichtung auf den Mikrokügelchen mit einer Säure, bevorzugt eine Carbonsäure oder ein Phenol.

4. Zusammensetzung nach einem der Ansprüche 1 bis 3, worin die Glasmikrokügelchen:
(a) zusätzlich zu dem Zinkoxid mindestens ein weiteres Bakterizid haben;
(b) einen Durchmesser von 5 bis 150 Mikrometer haben; oder
(c) einen Durchmesser von 10 bis 50 Mikrometer haben.

5. Zusammensetzung nach einem der Ansprüche 1 bis 4, worin das Zinkoxid oder Zinksalz in der Beschichtung in Partikelclustern eines Durchmessers von 0,3 bis 0,5 Mikrometern vorliegen und worin die Partikel vorzugsweise weitgehend transparent sind.

6. Zusammensetzung nach einem der Ansprüche 1 bis 5, worin der Träger ein dermatologisch zulässiges Vehikel ist, das vorzugsweise einen mehrwertigen Alkohol aufweist, ein Silicon, Ethanol, Wasser oder eine beliebige Kombination von zwei oder mehreren davon.

7. Zusammensetzung nach Anspruch 6, die 0,01% bis 10 Gewichtsprozent der Partikel enthält.

8. Zusammensetzung nach einem der vorgenannten Ansprüche, die in Form einer Aerosol-, Roller-, Gel-, Stift-, Creme-, Lotion- oder Pumpspray-Zubereitung vorliegt.

9. Verwendung von Partikeln, aufweisend, Glasmikrokügelchen, die darauf aufgesintert eine Beschichtung aus Zinkoxid hat, aus einem Zinksalz oder einer beliebigen Mischung von zwei oder mehreren davon, als ein Deodorant in Zusammensetzungen zum topischen Auftrag.

10. Verwendung nach Anspruch 9, worin die Partikel wie in einem der Ansprüche 2 bis 5 festgelegt sind.

11. Verfahren zum Verhüten oder Vermindern von üblem Körpergeruch, welches Verfahren das Auftragen einer Deodorantzusammensetzung nach einem der Ansprüche 1 bis 8 auf die Haut umfasst

12. Verfahren zum Beschichten von Glasmikrokügelchen mit Zinkoxid, welches Verfahren das Erhitzen einer bewegten Mischung von Glasmikrokügelchen und einer Zinkverbindung in einem flüssigen Reaktionsmittel umfasst, um eine milchige Suspension von kolloidalem Zinkoxid zu erzeugen, und weiteres Erhitzen der Suspension bis 190° bis 200°C, um das Zinkoxid als eine haftende Beschichtung abzuscheiden, die auf den Glasmikrokügelchen aufgesintert ist.

13. Verfahren nach Anspruch 12, worin
(a) die Mischung bis 140° bis 160°C zur Erzeugung der milchigen Suspension erhitzt wird;
(b) das flüssige Reaktionsmittel ein Glykol aufweist, vorzugsweise Diethylenglykol oder Polyethylenglykol; und/oder
(c) die Mischung für 1 bis 3 Stunden zur Erzeugung der Suspension erhitzt wird und für weitere 1 bis 3 Stunden, um die Beschichtung abzuscheiden.

14. Verfahren zum Beschichten von Glasmikrokügelchen mit einem Zinksalz, welches Verfahren das Beschichten der Glasmikrokügelchen mit Zinkoxid mit Hilfe des Verfahrens nach einem Ansprüche 12 und 13 umfasst, und anschließend Kontaktieren der Beschichtung mit einer Säure, um darauf ein Zinksalz zu erzeugen.

15. Verfahren nach Anspruch 14, bei welchem
(a) lediglich ein Teil der Zinkoxid-Beschichtung in ein Zinksalz umgewandelt wird;
(b) die Säure eine Carbonsäure oder ein Phenol ist;
(c) die Säure eine Carbonsäure ist, die Pidolinsäure ist, Essigsäure, Euginol, Pyrrolidon-S-carbonsäure, Zimtsäure, Citronensäure oder Glycin; und/oder
(d) nach dem Erzeugen des Zinksalzes auf den Mikrokügelchen diese mit Wasser ohne Entfernung des Salzes gewaschen werde.

## Revendications

1. Composition désodorisante pour une application topique, qui comprend une pluralité de particules comprenant chacune une microsphère en verre au moins partiellement revêtue avec un composé de zinc fritté sur la microsphère en verre, le composé de zinc étant choisi parmi l'oxyde de zinc, un sel de zinc ou tout mélange de deux de ceux-ci ou plus, lesdites particules étant dispersées dans un transporteur.

2. Composition selon la revendication 1, dans laquelle le revêtement comprend de l'oxyde de zinc et un sel de zinc, de préférence le sel de zinc est le pidolate de zinc, l'acétate de zinc, l'eugolinate de zinc, le pyrrolidone-5-carboxylate de zinc, le cinnamate de zinc, le citrate de zinc ou le glycinate de zinc.

3. Composition selon la revendication 1 ou 2, dans laquelle le sel de zinc est formé en mettant en contact un revêtement d'oxyde de zinc sur les microsphères avec un acide, de préférence un acide carboxylique ou un phénol.

4. Composition selon l'une quelconque des revendications 1 à 3, dans laquelle les microsphères en verre :
(a) ont au moins un autre bactéricide en plus de l'oxyde de zinc ;
(b) ont un diamètre de 5 à 150 microns ; ou
(c) ont un diamètre de 10 à 50 microns.

5. Composition selon l'une quelconque des revendications 1 à 4, dans laquelle l'oxyde de zinc ou le sel de zinc est présent dans le revêtement en groupements de particules d'un diamètre de 0,3 à 0,5 micron, lesdites particules étant de préférence essentiellement transparentes.

6. Composition selon l'une quelconque des revendications 1 à 5, dans laquelle le transporteur est un véhicule dermatologiquement acceptable, qui comprend de préférence un alcool polyhydrique, une silicone, de l'éthanol, de l'eau ou toute combinaison de deux de ceux-ci ou plus.

7. Composition selon la revendication 6, qui contient de 0,01 à 10 % en poids desdites particules.

8. Composition selon l'une quelconque des revendications précédentes, qui se présente sous la forme d'une formulation d'aérosol, de roll-on, de gel, de bâton, de crème, de lotion ou de pulvérisation à pompe.

9. Utilisation de particules comprenant des microsphères en verre sur lesquelles est fritté un revêtement d'oxyde de zinc, un sel de zinc ou tout mélange de deux de ceux-ci ou plus, comme déodorant dans des compositions pour une application topique.

10. Utilisation selon la revendication 9, dans laquelle les particules sont telles que définies dans l'une quelconque des revendications 2 à 5.

11. Procédé de prévention ou de réduction des mauvaises odeurs qui consiste à appliquer sur la peau une composition désodorisante selon l'une quelconque des revendications 1 à 8.

12. Procédé de revêtement de microsphères en verre avec de l'oxyde de zinc, lequel procédé consiste à chauffer un mélange agité de microsphères en verre, et un composé de zinc dans un milieu réactionnel liquide, pour former une suspension laiteuse d'oxyde de zinc colloïdal, et en outre à chauffer la suspension à une température allant de 190 °C à 200 °C pour déposer l'oxyde de zinc comme un revêtement adhérent fritté sur les microsphères en verre.

13. Procédé selon la revendication 12, dans lequel :
(a) le mélange est chauffé à une température allant de 140 °C à 160 °C pour former ladite suspension laiteuse ;
(b) le milieu réactionnel liquide comprend un glycol, de préférence du diéthylène glycol ou du poly(éthylène glycol) ; et/ou
(c) le mélange est chauffé pendant 1 à 3 heures pour former ladite suspension, et pendant encore 1 à 3 heures pour déposer ledit revêtement.

14. Procédé de revêtement de microsphères en verre avec un sel de zinc qui consiste à revêtir les microsphères en verre avec de l'oxyde de zinc par le procédé selon l'une quelconque des revendications 12 et 13, puis à mettre en contact le revêtement avec un acide pour former dessus un sel de zinc.

15. Procédé selon la revendication 14, dans lequel :
(a) seulement une partie du revêtement d'oxyde de zinc est convertie en un sel de zinc ;
(b) l'acide est un acide carboxylique ou un phénol ;
(c) l'acide est un acide carboxylique qui est l'acide pidolique, l'acide acétique, l'euginol, l'acide pyrrolidone-S-carboxylique, l'acide cinnamique, l'acide citrique, ou la glycine ; et/ou
(d) après formation du sel de zinc sur les microsphères, elles sont lavées avec de l'eau sans élimination du sel.
